Europäisches Patentamt

European Patent Office

Office européen des brevets

(11) Veröffentlichungsnummer: **0 145 893**
**A1**

(12) ## EUROPÄISCHE PATENTANMELDUNG

(21) Anmeldenummer: **84112722.8**

(22) Anmeldetag: **22.10.84**

(51) Int. Cl.⁴: **A 61 B 6/04,** A 61 B 6/10,
**B 23 Q 15/24**

(30) Priorität: **04.11.83 DE 3340019**

(43) Veröffentlichungstag der Anmeldung: **26.06.85**
**Patentblatt 85/26**

(84) Benannte Vertragsstaaten: **DE FR**

(71) Anmelder: **Siemens Aktiengesellschaft, Berlin und
München Wittelsbacherplatz 2, D-8000 München 2 (DE)**

(72) Erfinder: **Summ, Herbert, Erlanger Strasse 8,
D-8531 Wilhelmsdorf (DE)**

(54) **Kompressionsvorrichtung für ein Röntgendiagnostikgerät.**

(57) Die Erfindung betrifft eine Kompressionsvorrichtung für
ein Röntgendiagnostikgerät mit einem durch einen Elektromotor (15a) verstellbaren Kompressionswagen (8), der einen Kompressionstubus trägt, dem Schaltmittel (29) zugeordnet sind, die den Motor abschalten, wenn der Kompressionstubus auf dem Aufnahmeobjekt auftrifft. Die Verbindung zwischen Motor (15a) und Kompressionstubus kann
gelöst werden (Übertragungsmittel 17, 17a).

Siemens Aktiengesellschaft       Unser Zeichen
Berlin und München               VPA 83 P 3379  E

Kompressionsvorrichtung für ein Röntgendiagnostikgerät

Die Erfindung betrifft eine Kompressionsvorrichtung für ein Röntgendiagnostikgerät mit einem durch einen Elektromotor verstellbaren Kompressionswagen, der einen Kompressionstubus trägt, dem Schaltmittel zugeordnet sind, die den Motor abschalten, wenn der Kompressionstubus auf dem Aufnahmeobjekt auftrifft.

Es ist ein Röntgendiagnostikgerät mit einem Kompressionswagen dieser Art bekannt, das zur Anfertigung von Aufnahmen der weiblichen Brust dient. Dabei wird das Aufnahmeobjekt auf eine Aufnahmeplatte aufgelegt und anschliessend motorisch komprimiert. Der Motor für den Kompressionswagen wird automatisch durch federbelastete Schaltmittel abgeschaltet, wenn die Kraft auf das Aufnahmeobjekt einen vorbestimmten Wert erreicht.

Bei der bekannten Kompressionsvorrichtung erfolgt eine ruckartige, also keine feinfühlige Abschaltung des Motors für die Kompressionsvorrichtung. Infolge der Trägheit der bewegten Teile ist es dabei möglich, daß die Kraft auf das Aufnahmeobjekt nach dem Abschalten des Motors in unerwünschter Weise noch weiter ansteigt.

Durch die DE-OS 23 56 276 ist es bei einem Röntgenzielgerät bereits bekannt, den Antriebsmotor in seiner Geschwindigkeit in Abhängigkeit von der auf den Patienten einwirkenden Kraft schrittweise umzuschalten.

Der Erfindung liegt die Aufgabe zugrunde, eine Kompres-

Tp 2 Ler / 12.03.1984

sionsvorrichtung der eingangs genannten Art so auszubilden, daß der Druck des Kompressionstubus auf das Aufnahmeobjekt von Hand gelöst werden kann.

Diese Aufgabe ist erfindungsgemäß dadurch gelöst, daß Mittel zum Lösen der Verbindung zwischen Motor und Kompressionstubus vorhanden sind. Bei der erfindungsgemäßen Kompressionsvorrichtung ist es möglich, den Motor von Hand vom Kompressionstubus abzukuppeln und damit den Kompressionstubus vom Aufnahmeobjekt von Hand zu lösen.

Zweckmäßige Ausgestaltungen der Erfindung ergeben sich aus den Unteransprüchen in Verbindung mit in den Zeichnungen dargestellten Ausführungsbeispielen. Es zeigen:

Fig. 1 ein Röntgendiagnostikgerät mit einer Kompressionsvorrichtung zur Erläuterung des Erfindungsgedankens,

Fig. 2 eine Einzelheit des Röntgendiagnostikgerätes gemäß Figur 1,

Fig. 3 eine Variante der Einzelheit gemäß Figur 2, und

Fig. 4 die Variante gemäß Figur 3 in einer anderen Ansicht.

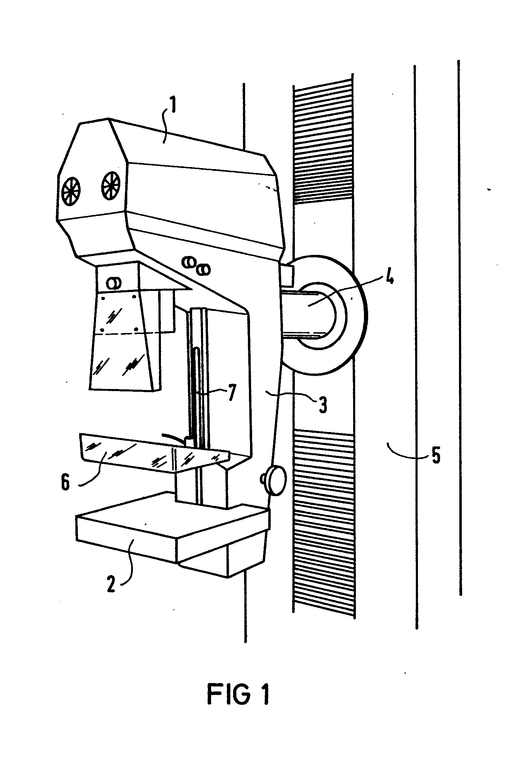

In der Figur 1 ist ein für Mammographien dienendes Röntgendiagnostikgerät dargestellt, das in einem Gehäuse 1 eine Röntgenröhre und eine Aufnahmeplatte 2 für das Aufnahmeobjekt aufweist. Die Komponenten 1, 2 sind an einem Halter 3 angeordnet, der mit Hilfe einer Achse 4 an einem Stativ 5 gelagert ist. Er ist relativ zum Stativ 5 um die Achse 4 dreh- sowie höhenverstellbar.

Zur Kompression des Aufnahmeobjektes ist eine Kompressionsvorrichtung mit einem Kompressionstubus 6 vorgesehen, der mit einem nicht dargestellten Ansatz in einen Schlitz 7 des Halters 3 höhenverstellbar ist. Er steckt mit diesem Ansatz in einem nachfolgend noch näher erläuterter Kompressionswagen, der durch einen Elektromotor verschiebbar ist. Auf diese Weise kann der Kompressionstubus 6 von oben gegen das auf der Aufnahmeplatte 2 liegende Aufnahmeobjekt gedrückt werden und dieses motorisch komprimieren.

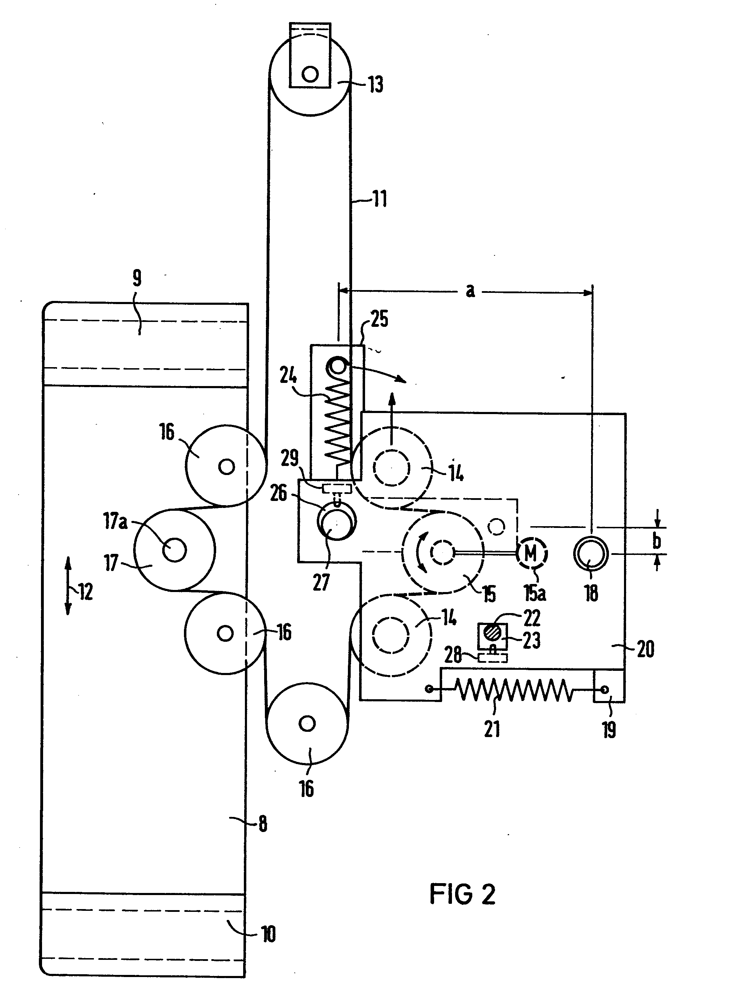

Die Figur 2 zeigt den Kompressionswagen 8 mit zwei Öffnungen 9, 10 für den Ansatz des Kompressionstubus 6. Der Kompressionstubus 6 wird dabei in die Öffnung 10 gesteckt, wenn Aufnahmen in der geschilderten Weise angefertigt werden. Soll eine Aufnahme in Vergrößerungstechnik angefertigt werden, so wird der Kompressionstubus 6 in die Öffnung 9 gesteckt und zwischen die Aufnahmeplatte 2 und den Kompressionstubus 6 eine Auflageplatte am Halter 3 im Abstand von der Aufnahmeplatte 2 befestigt. In diesem Fall erhält man dann vergrößerte Röntgenaufnahmen.

Der Kompressionswagen 8 wird durch einen Zahnriemen 11 in Richtung des Pfeiles 12 verstellt, der über eine Spannrolle 13, zwei Rollen 14, eine Antriebsrolle 15, Führungsrollen 16 sowie eine Kupplungsrolle 17 geführt ist. Die Antriebsrolle 15 ist gerätfest und wird durch einen Elektromotor 15a angetrieben. Sie liegt hinter zwei auf einer gerätfesten Achse 18 schwenkbar gelagerten Platten 19, 20, von denen die Platte 19 auf ihrer Rückseite die Rollen 14 trägt. Die Platte 20 ist mit Hilfe einer Feder 21 mit der Oberkante einer Öffnung 23 gegen einen Anschlag 22 an der Platte 19 gedrückt. Die Platte 20 ist durch eine stärkere Feder 24, die einerseits an einem Hebel 25 und andererseits an der Platte 20 angreift, mit der Unterseite einer Öffnung 26 gegen einen gerätfesten Anschlag 27 gedrückt.

Zur Verstellung des Kompressionswagens 8 wird die Kupplungsrolle 17 auf ihrer Achse 17a arretiert, so daß beim Drehen der Antriebsrolle 15 eine Auf- bzw. Abbewegung erfolgt. Die Aufbewegung erfolgt dabei immer mit derselben Geschwindigkeit. Die Abbewegung erfolgt zunächst mit einer hohen Geschwindigkeit, bis der in der Figur 2 nicht dargestellte Kompressionstubus am Aufnahmeobjekt anliegt. Durch den auftretenden Widerstand erhöht sich der Zug im Zahnriemen 11 und die Rollen 14 bewegen sich zusammen mit der Platte 19 um die Achse 18 entgegen dem Uhrzeigersinn. Dabei wird die Feder 21 gespannt. Erreicht bei dieser Bewegung der Anschlag 22 die Unterkante der Öffnung 23, so betätigt er einen Mikroschalter 28 und der Antriebsmotor 15a und somit der Kompressionswagen 8 wird auf eine niedrigere Geschwindigkeit umgeschaltet.

Bei der Weiterbewegung des Kompressionstubus und damit auch des Kompressionswagens 8 nimmt nun die weiter durch den Rollen 14 entgegen dem Uhrzeigersinn verschwenkte Platte 19 mit dem Anschlag 22 die Platte 20 an der Unterkante der Öffnung 23 mit, so daß auch die Platte 20 entgegen dem Uhrzeigersinn verschwenkt wird, wobei die Feder 24, die härter ist als die Feder 21, gespannt wird. Dabei bewegt sich die Öffnung 26 relativ zum Anschlag 27, und zwar so weit, bis der Anschlag 27 an einem an der Oberseite der Öffnung 26 angeordneten Mikroschalter 29 anschlägt und den Antriebsmotor 15a stillsetzt.

Die Feder 24 ist zusammen mit dem Hebel 25 auf der Achse 27 im Uhrzeigersinn verschwenkbar. Bei dieser Schwenkbewegung verkleinert sich der Hebelarm. In Figur 2 sind beispielsweise ein großer Hebelarm a und ein kleiner Hebelarm b für die Federkraft eingetragen. Demgemäß ist durch Verschwenkung des Hebels 25 die Kraft, bei der die Endabschaltung des Antriebsmotors 15a erfolgt, einstellbar.

Soll der Kompressionstubus 6 mit dem Kompressionswagen 8 von Hand verstellt werden, so kann die Kupplung zwischen der Welle 17a und der Kupplungsrolle 17 von Hand gelöst werden, so daß sich die Kupplungsrolle 17 auf der Welle 17a frei drehen kann.

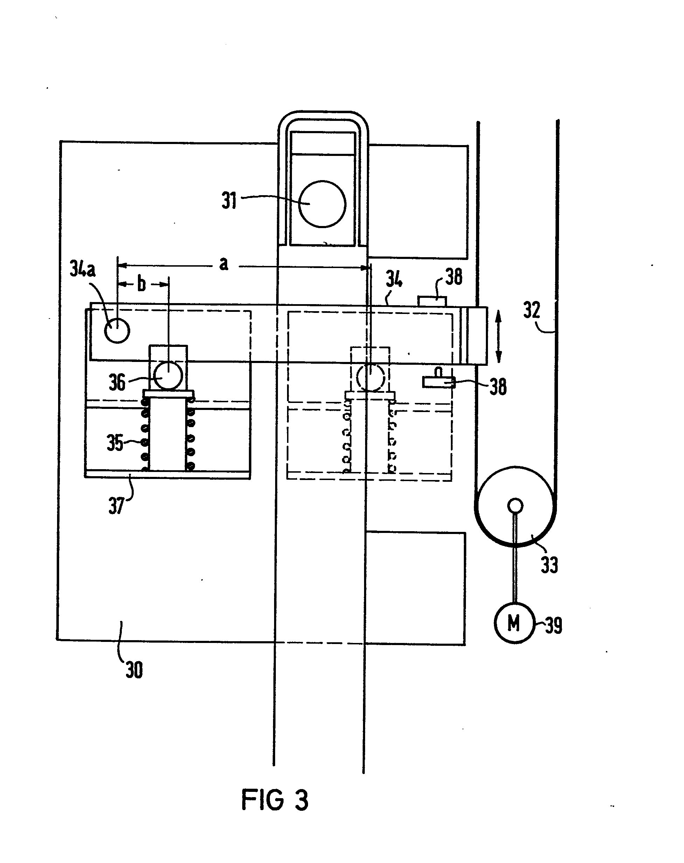

Das Ausführungsbeispiel gemäß Figur 3 zeigt einen Kompressionswagen 30, der im Gegensatz zum Kompressionswagen 8 der Figur 2 in einer Ansicht von vorne dargestellt ist. Eine obere Öffnung 31 dient zur Aufnahme des Kompressionstubus 6 für die Anfertigung einer Aufnahnme in Vergrößerungstechnik. Entsprechend der Figur 2 ist auch eine nicht dargestellte, untere Öffnung für normale Aufnahmen vorgesehen. Zur Verstellung des Kompressionswagens 30 dient ein Zahnriemen 32, der über eine Rolle 33 geführt und an einem Hebel 34 befestigt ist, der um eine Achse 34a auf dem Kompressionswagen 30 schwenkbar ist.

Der Hebel 34 wird durch eine Feder 35, die einen Anschlag 36 gegen die Unterkante des Hebels 34 drückt und sich andererseits an einem Federwagen 37 abstützt, gegen einen Anschlag 38 gedrückt. Der Federwagen 37 ist in horizontaler Richtung auf dem Kompressionswagen 30 in nicht dargestellten Führungen verstellbar. Seine vertikale Lage auf dem Kompressionswagen 30 ist dagegen nicht veränderbar.

Stößt der Kompressionstubus am Aufnahmeobjekt an, so bewegt sich der Zahnriemen 32 mit dem rechten Ende des Hebel 34 weiter nach unten, bis er an einem Mikroschalter 38 anstößt und den Motor 39 stillsetzt. Die Kraft, bei der der Motor 60, der die Rolle 33 antreibt, stillgesetzt wird, ist dabei durch Horizontalverschiebung des Federwagens 37 einstellbar. In der gestrichelt angestellten Stellung des Federwagens 37 ist diese Kraft größer als in der voll ausgezogen gezeichneten Stellung.

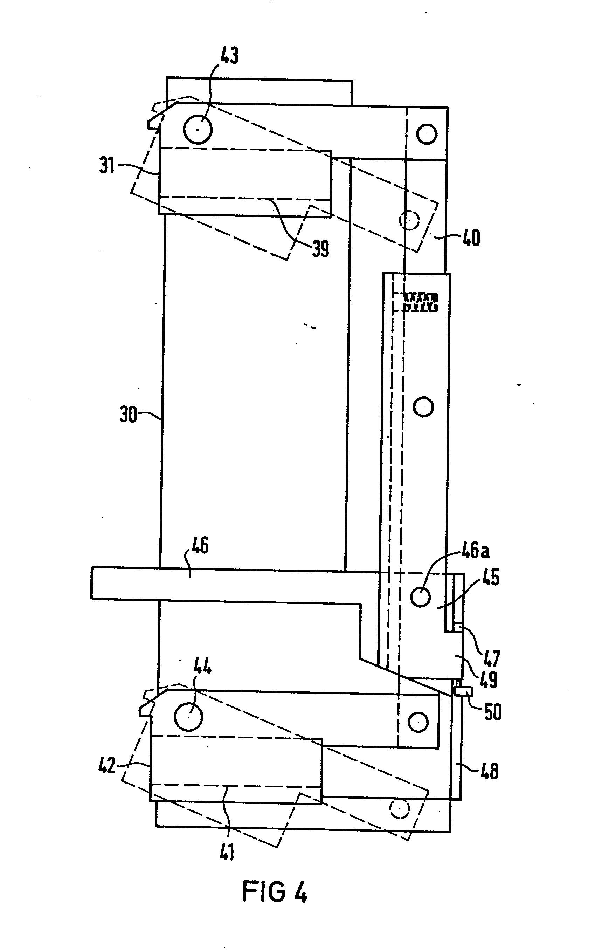

Die Umschaltung des Antriebsmotors 60 auf niedrigere Geschwindigkeit ist in Verbindung mit der Figur 4 näher erläutert, die den Kompressionswagen 30 in einer Seitenansicht zeigt. Aus der Figur 4 geht hervor, daß die Öffnung 31 von einer Hülse 39 gebildet wird, die über eine Stange 40 mit einer darunter liegenden Hülse 41 mit einer Öffnung 42 gelenkig verbunden ist. Die Hülse 41 dient dabei zur Aufnahme des Kompressionstubus 6 für normale Röntgenaufnahmen. Die Hülsen 39 und 41 sind um Achsen 43, 44 schwenkbar. An der Stange 40 ist ein Riegel 45 verstellbar gelagert, der mit Hilfe eines Hebels 46 aus einer Öffnung 47 einer Arretierplatte 48 herausbewegbar ist.

In der voll gezeichneten Stellung der Hülsen 39, 41 liegt der Ansatz 49 des Riegels 45 in der Öffnung 47. Er ist so ausgebildet, daß zwischen dem Ansatz 49 und der Öffnung 47 ein Spiel vorhanden ist. Aufgrund des Tubusgewichtes und der Federkraft wird dabei der Ansatz 49 gegen die Oberkante der Öffnung 47 gedrückt, wenn der Tubus 6 noch nicht am Aufnahmeobjekt anliegt. Erreicht er das Aufnahmeobjekt, so werden die Hülsen 39, 41 ganz geringfügig im Uhrzeigersinn verschwenkt und der Ansatz 49 bewegt sich in die in der Figur 4 gezeichnete untere Stellung, in der er einen Mikroschalter 50 betätigt und den Motor 39 auf geringere Geschwindigkeit umschaltet.

Für die Freigabe des Aufnahmeobjektes von Hand dient der Hebel 46. Wird dieser um die Achse 46a im Uhrzeigersinn verschwenkt, so zieht er den Riegel 45 mit dem Ansatz 49 aus der Öffnung 47, so daß die Hülsen 39, 41 von Hand in die gestrichelt gezeichnete Lage verstellt werden können, in der der Tubus 6 über seiner Endstellung liegt und das Aufnahmeobjekt freigibt.

3 Patentansprüche
4 Figuren

Patentansprüche

1. Kompressionsvorrichtung für ein Röntgendiagnostikgerät mit einem durch einen Elektromotor (15a, 39) verstellbaren Kompressionswagen (8, 30), der einen Kompressionstubus (6) trägt, dem Schaltmittel (29, 38) zugeordnet sind, die den Motor (15a, 39) abschalten, wenn der Kompressionstubus (6) auf dem Aufnahmeobjekt auftrifft, d a d u r c h   g e k e n n z e i c h n e t ,   daß Mittel (17, 17a, 39, 41, 45, 46) zum Lösen der Verbindung zwischen Motor (15a, 60) und Kompressionstubus (6) vorhanden sind.

2. Kompressionsvorrichtung nach Anspruch 1,   d a - d u r c h   g e k e n n z e i c h n e t ,   daß Mittel (17, 17a) zum Lösen der Verbindung zwischen Motor (15a) und Kompressionswagen (8) vorgesehen sind.

3. Kompressionsvorrichtung nach einem der Ansprüche 1 und 2,   d a d u r c h   g e k e n n z e i c h n e t , daß am Kompressionswagen (30) Halterungsmittel (39, 41) für den Kompressionstubus (6) vorgesehen sind, die derart um eine horizontale Achse (43, 44) verdrehbar sind, daß der Kompressionstubus beim Lösen einer Sperre (45, 46) von Hand um einen vorbestimmten Weg nach oben verstellbar ist.

0145893

FIG 1

0145893

83 P 3379

FIG 2

FIG 3

FIG 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | DE-A-2 356 276 (VEB TRANSFORMA-TOREN- UND RÖNTGENWERK HERMANN MATERN) <br><br> * Seite 3, Zeilen 16-29; Seite 5, Zeilen 2-9; Seite 10, Zeilen 3-28; Abbildung 3 * | 1 | A 61 B 6/04 <br> A 61 B 6/10 <br> B 23 Q 15/24 |
| A | | 5 | |
| | --- | | |
| Y | US-A-4 031 442 (J. POPPELREITER) <br><br> * Zusammenfassung; Spalte 2, Zeilen 13-49; Spalte 3, Zeilen 50-68; Spalte 5, Zeilen 37-68; Spalte 6, Zeilen 5-21; Abbildungen 1-4 * | 1 | |
| | --- | | **RECHERCHIERTE SACHGEBIETE (Int. Cl.4)** |
| A | DE-A-1 942 490 (SIEMENS AG) <br><br> * Seite 2, Zeilen 7-28; Seite 3, Zeilen 18-32; Seite 4, Zeilen 3-24; Abbildung * | 1,2,5, 6 | A 61 B <br> B 23 Q |
| | --- | | |
| A | DE-A-2 631 102 (SIEMENS AG) <br><br> * Seite 5, Zeilen 3-21; Seite 7, Zeilen 15-28; Abbildung * | 1 | |
| | --- | | |
| | -/- | | |

Der vorliegende Recherchenbericht wurde für alle Patentansprüche erstellt.

| Recherchenort | Abschlußdatum der Recherche | Prüfer |
|---|---|---|
| DEN HAAG | 14-02-1985 | RIEB D. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein-stimmendes Dokument

EPA Form 1503 03 82

Europäisches
Patentamt

**EUROPÄISCHER RECHERCHENBERICHT**

0145893
Nummer der Anmeldung

EP 84 11 2722

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB-A-1 041 424 (GENERAL ELECTRIC CO.LTD.) <br><br> * Seite 1, Zeilen 65-74; Seite 2, Zeilen 119-129; Seite 3, Zeilen 63-73; Abbildungen 1-4 * <br><br> --- | 1,4 | |
| A | FR-A-1 240 117 (G. MASSIOT & CIE) <br><br> * Seite 3, linke Spalte, Zeilen 29-43; Seite 3, rechte Spalte, Zeilen 51 - Seite 4, linke Spalte, Zeile 11; Abbildungen 4,5 * <br><br> ----- | 1,5 | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| DEN HAAG | 14-02-1985 | RIEB D. |